# EUROPEAN PATENT SPECIFICATION

(11) **EP 2 457 562 B1**
(45) Date of publication and mention of the grant of the patent: **05.04.2017**
(21) Application number: 12156178.1
(22) Date of filing: 09.05.2007
(51) Int. Cl.: A61K 9/24, A61K 9/36, A61K 31/4458

(54) **Zero-order modified release solid dosage forms**
Feste Darreichungsformen mit modifizierter Freisetzung nullter Ordnung
Formes posologiques solides à libération modifiée d'ordre zéro

(30) Priority: 09.05.2006 US 798889 P; 01.11.2006 US 856226 P
(43) Date of publication of application: 30.05.2012
(62) Divisional of application: 07776913.1
(73) Proprietor: Mallinckrodt LLC, Hazelwood, MO 63042 (US)
(72) Inventor: Rastogi, Suneel Kumar, Ballwin, Missouri 63021 (US); Meadows, Justin Clark, St. Louis, Missouri 63119 (US); Gupta, Vishal Kumar, Ballwin, Missouri 63021 (US)
(74) Representative: Jones, Nicholas Andrew

(56) References cited:
- WO-A-03/063834
- US-A- 4 610 870
- US-B1- 6 500 459

## Description

### BACKGROUND OF THE INVENTION

### Field of the Invention

The present invention relates to modified release solid dosage forms suitable for administration of a wide range of water-soluble pharmaceutical agents at a zero-order release rate, and to a process of making the same. More specifically, the invention relates to modified release tablets having a matrix core containing methylphenidate hydrochloride surrounded by a modified release coating.

### Description of the Related Art

Modified release dosage forms, which are well known in the art, release their drug content gradually and over an extended period of time after the drug makes contact with an aqueous solution (e.g., *in-vitro* dissolution), or gastrointestinal fluid (*in-vivo*). These dosage forms are desirable in treating various diseases because the desired drug concentration is maintained *in-vivo* for longer periods of time as compared to immediate release dosage forms, allowing for less frequent dosing.

For many extended release formulations, the rate of drug release initially increases rapidly followed by a decreased rate of drug release. This type of drug release is categorized as first-order release. Such formulations may not produce uniform concentration levels of the drug in the bloodstream for a prolonged period of time.

Zero-order release dosage forms are also known in the art. The term "zero-order release," "zero-order dissolution" or "zero-order rate" refers to a rate of release of a drug from the solid dosage form after coming in contact with an aqueous environment, which is uniform or nearly uniform independent of the drug concentration in the dosage form during a given time period. Zero-order release dosage forms usually enable reduced dosing frequency compared to less sustained or more unevenly released dosage forms, thus improving patient compliance. Dosage forms with zero-order release generally provide maximum therapeutic value with minimal side effects. Although such dosage forms are advantageous, it has been difficult to formulate highly water soluble pharmaceutical agents in such a dosage form because the agents are susceptible to a phenomenon known as "dose dumping". The dosage form can release the drug rapidly at a high concentration, effectively "dumping" the drug into the bloodstream and potentially overdosing the patient.

Coated matrix systems have been devised in an effort to achieve zero-order release of various active agents. Woodall et al., "Matrix Tablets Containing a Zero Order Dissolution Profile," The AAPS Journal, Vol. 7, No.2, Abstract W5253 (2005) report zero order kinetics for a compressed matrix tablet coated with a 3% or 5% weight gain of ethyl cellulose. Porter et al., "Modified Release Matrix Tablets Coated with a Modified-Release Film Coating: Comparison of Regular Tablets with a Mini-tablet Multiparticulate System," AAPS PharmSci, Vol. 4, No. 4, Abstract T3352 (2002) describe matrix cores containing chlorpheniramine maleate and HPMC, coated with an aqueous ethyl cellulose dispersion (SURELEASE™), modified with a water-soluble polymer system (OPADRY™), which are reported to exhibit zero-order kinetics. US6500459 describes a pharmaceutical composition comprising: (i) a core comprising: (a) an active ingredient; (b) a hydrophilic carrier; (c) a hydrodynamic diffusion enhancer; and optionally (d) conventional pharmaceutically acceptable excipients; and (ii) a functional coating membrane surrounding said core.

The matrix core of a modified release dosage form is often prepared by a wet granulation process. Wet granulation can involve milling of drugs and other ingredients, preparing a binder, mixing the binder with the milled ingredients to form a wet mass, coarse screening of the wet mass, drying of the wet mass to form granules, screening of granules, mixing the screened granules with lubricants or other excipients, extruding the mixture, and compressing to form a solid dosage form. Wet granulation is an expensive, time consuming process requiring many processing steps and significant capital equipment.

U.S. Patent No. 6,673,367 reports controlled release of methylphenidate from beads coated with a sustained release coating produced by various methods of granulation. Alternatively, methylphenidate HCl is mixed with Lactose DT, Methocel, talc, magnesium stearate and optionally Eudragit L 100-55 and directly compressed into an uncoated tablet.

Methylphenidate and methylphenidate hydrochloride are psychostimulants prescribed for the treatment of Attention Deficit Disorder (ADD), the most common psychiatric disorder in children. The disorder is characterized by inattentiveness and impulsiveness and may be present with hyperactivity (ADHD) as well as cognitive and learning problems. The mechanism of action of methylphenidate is believed to involve blocking the uptake of increased levels of extracellular dopamine and norepinephrine at nerve terminal transporters. The drug is a mild stimulant of the central nervous system with more prominent effects on mental activities than motor skills.

It has been reported that conventional forms of methylphenidate exhibit peak blood levels at 1 to 3 hours and have a half-life from 2 to 4 hours in adults and children. Methylphenidate formulations with immediate release characteristics, such as Ritalin™, are typically administered in the morning and afternoon to ensure that a child receives an adequate dose for an 8 to 12 hour period. This requires dosing during the school day, which is preferably avoided to improve patient compliance and potential dosing errors. Patient compliance can be a problem in schools which prohibit children from taking medications during the school day or require administration only during hours when a nurse is present. Also, the rapid release of the drug into the bloodstream results in a maximum dosage for a brief period of the day with declining dosage thereafter. A substantially uniform release of the drug is more preferable so that the drug affects the child essentially consistently throughout the day.

A sustained release form of methylphenidate (Ritalin™ SR) is commercially available, but has been reported to be less effective in early morning behavior management and no more effective than immediate release dosage forms such as Ritalin™.

Adverse side effects of methylphenidate use include hypertension, tachycardia, angina, arthralgia, dyskinesia, fever, skin rash or hives, thrombocytopenia, blurred vision or other changes in vision, convulsions, muscle cramps, Tourette's syndrome, toxic psychosis, or weight loss. Long-term effects of methylphenidate in children are not well established. Thus, it is desirable to maintain a more consistent plasma concentration in the bloodstream over the course of a school day to avoid such side effects. It is also desirable to limit the use of the drug to the hours in which it is most needed to further limit the risk of side effects.

There is a need for a zero-order release oral dosage form of methylphenidate hydrochloride or other water soluble drugs with a 6 to 10 hour release of drug for administration once per day.

### SUMMARY OF INVENTION

Briefly, the present invention is directed to a solid dosage form comprising a matrix core, a modified release coating and a water-soluble barrier coating. The matrix core comprises between about 10% and about 50% of a non-swellable hydrophobic material comprising at least one polymer and a water-soluble pharmaceutical agent. The modified release coating surrounds the matrix core, and comprises a hydrophobic polymer and a hydrophilic pore-forming agent. The water-soluble barrier coating is between the modified release coating and the matrix core such that the barrier coating surrounds the matrix core and the modified release coating surrounds the barrier coating. The solid dosage form is capable of releasing the pharmaceutical agent at a zero-order rate for a period of at least four hours after administration to a subject.

Another aspect of the invention is directed to a method of making a solid dosage form by mixing between about 10% and about 50% of a non-swellable hydrophobic material comprising at least one polymer, from about 2 to 25 wt % of a water-soluble pharmaceutical agent, an optional filler, an optional lubricant, and an optional glidant to form a mixture, compressing the mixture to form a matrix core, coating the matrix core to form a barrier coating surrounding the matrix core, drying the barrier coating, coating the barrier-coated matrix core with a modified release coating, and drying the modified release coating to form the solid dosage form. The barrier coating comprises a water-soluble polymer, and the modified release coating comprises a hydrophobic polymer and a hydrophilic pore-forming agent. The solid dosage form is capable of releasing the pharmaceutical agent at a zero order rate for a period of at least four hours after administration to a subject.

### BRIEF DESCRIPTION OF THE DRAWINGS

Figure 1A is a graph depicting the percentage of methylphenidate hydrochloride dissolved as a function of time for uncoated matrix core tablets, as described in example 1.
Figure 1B is a plot of In (100-%methylphenidate hydrochloride dissolved) as a function of time indicating first order drug release for the uncoated tablets.
Figure 2A is a graph depicting the percentage of methylphenidate hydrochloride dissolved as a function of time for (ethyl cellulose:hypromellose - 70:30) coated tablets, as described in example 1.
Figure 2B is a plot of In (100-%methylphenidate hydrochloride dissolved) as a function of time indicating zero order drug release for the (ethyl cellulose:hypromellose - 70:30) coated tablets.
Figure 3 is a graph depicting the percentage of methylphenidate hydrochloride dissolved as a function of time, as described in example 2 for uncoated matrix core tablets.
Figure 4A is a graph of the percentage of methylphenidate hydrochloride dissolved as a function of time, as described in example 2 for (ethyl cellulose:hypromellose - 70:30) coated tablets containing glyceryl behenate.
Figure 4B is a plot of ln (100-%methylphenidate hydrochloride dissolved) as a function of time and indicates zero order drug release for the 6% and 8% coated tablets containing glyceryl behenate.
Figure 5A is a graph of the percentage of methylphenidate hydrochloride dissolved as a function of time, as described in example 3 for the (ethyl cellulose:hypromellose - 80:20) coated tablets.
Figure 5B is a plot of In (100-%methylphenidate hydrochloride dissolved) as a function of time indicating zero order drug release for the (ethyl cellulose:hypromellose - 80:20) 4% modified release coated tablets.
Figure 6 is a graph of the percentage of methylphenidate hydrochloride dissolved as a function of time, as described in example 4 for the 95:5 ethyl cellulose:hypromellose coated tablets.
Figures 7 and 8 are graphs of the percentage of methylphenidate hydrochloride dissolved as a function of time, as described in example 5 for (ethyl cellulose:hypromellose - 70:30), 4% hypromellose seal coated tablets.
Figure 9 is a graph of the percentage of methylphenidate hydrochloride dissolved as a function of time, as described in example 5 for (ethyl cellulose:triacetin:hypromellose - 70:3.5:26.5), 4% hypromellose seal coated tablets.
Figures 10 and 11 are graphs of friability as a function of hardness for uncoated matrix core tablets as described in example 6, wherein 0.80% is the USP limit for 100 revolutions.
Figures 12 and 13 are graphs of the percentage of methylphenidate hydrochloride dissolved as a function of time, as described in example 6 for uncoated matrix core tablets of various hardness formed under various compression forces.
Figures 14 and 15 are graphs of the percentage of methylphenidate hydrochloride dissolved as a function of time, as described in example 8 for methylphenidate hydrochloride overcoated tablets.

### DETAILED DESCRIPTION OF THE INVENTION

The present invention provides for a modified release solid dosage form of a highly water soluble pharmaceutical ingredient, such as methylphenidate or a salt thereof, which exhibits zero-order kinetics upon dissolution. Such a dosage form eliminates the need for twice-a-day dosing, improves the likelihood of patient compliance, and establishes a substantially consistent drug dosage throughout its 6 to 10 hour period of effectiveness following administration. It also prevents or minimizes the risk of dose dumping and accidental overdose from improper dissolution of the dosage form. In the unlikely event that the coating on the dosage form ruptures, drug release from the core is still controlled by the hydrophobic material in the matrix. Moreover, the solid dosage form can be easily prepared under ambient conditions with minimal processing steps and equipment requirements.

The solid dosage form of the invention comprises a matrix core, a water-soluble barrier coating, and a modified release coating. The matrix core comprises between about 10% and about 50% of a non-swellable hydrophobic material comprising at least one polymer and a water-soluble pharmaceutically active agent, and optional excipients such as fillers, release modifiers, lubricants and glidants. The modified release coating is comprised of a hydrophobic polymer, a hydrophilic pore-forming agent, and an optional plasticizer. The term "pore-forming agents" includes materials that can be dissolved, extracted or leached from the modified release coating in the environment of use.

The solid dosage form includes a water-soluble barrier coating between the modified release coating and the matrix core such that the barrier coating surrounds the matrix core and the modified release coating surrounds the barrier coating. The barrier coating, also described herein as a seal coating, is comprised of a water soluble polymer and serves as a barrier between the pharmaceutically active agent in the matrix core and the modified release coating. It has been discovered that such a barrier can be effective in maintaining the integrity of the matrix tablet as well as the modified release coating on a tablet subjected to conventional storage conditions, as described further in example 3.

The solid dosage forms of the present invention provide for zero-order release of the pharmaceutical agent upon dissolution. When the dosage form is exposed to gastrointestinal or other fluids, the fluid diffuses through the modified release coating, dissolving the hydrophilic pore-forming agent to form pores or channels in the modified release coating. The fluid continues to diffuse through the barrier coating (if present) and matrix core, dissolving the pharmaceutical agent embedded in the matrix core. The pharmaceutical agent travels through the pores in the modified release coating out of the dosage form.

The matrix core includes a pharmaceutically active agent, a hydrophobic material, and optional fillers, release modifiers, lubricants, and glidants. The pharmaceutically active agent within the matrix core can be any water soluble pharmacologically active compound. Examples of such active agents suitable in the present invention include antihistamines, antibiotics, antituberculosis agents, cholinergic agents, antimuscarinics, sympathomimetics, sympatholytic agents, autonomic drugs, iron preparations, haemostatics, cardiac drugs, antihypertensive agents, vasodilators, non-steroidal antiinflammatory agents, opiate agonists, anticonvulsants, tranquilizers, stimulants, barbiturates, sedatives, expectorants, antiemetics, gastrointestinal drugs, heavy metal antagonists, antithyroid agents, genitourinary smooth muscle relaxants and vitamins. Suitable water soluble actives include, but are not limited to, abacavir sulfate, acyclovir, aminocaproic acid, alendronate sodium, amitriptyline hydrochloride, amphetamine, acetaminophen, allopurinol, amoxicillin, atenolol, atropine sulfate, azithromycin, balsalazide, benzepril hydrochloride, bisoprolol fumarate, bupropion hydrochloride, buformin, calacyclovir, captopril, cefprozil, cetrizine hydrochloride, cimetidine, ciprofloxacin, clindamycin, chlorpheniramine maleate, chlorpromazine hydrochloride, clomipramine hydrochloride, clonidine hydrochloride, clopidogrel bisulfate, cloxacillin sodium, codeine phosphate, colchicines, cyclophosphamide, diethylcarbamazine citrate, diltiazem. doxycycline, doxepin, DL-methionine, eprosartan, ethambutol hydrochloride, ethosuximide, erythromycin, fexofenadine, ferrous sulfate, fluoxetine hydrochloride, fluvastatin, fosonopril sodium, gabapentin, hydralazine hydrochloride, hydrocodone bitartrate, hydroxyzine hydrochloride, hydroxyurea, indinavir sulfate, isoniazid, isosorbide mononitrate, lactobionate, lamivudine, levamisole hydrochloride, levofloxacin, lisinopril, losartan potassium, metformin hydrochloride, methylphenidate, methylphenidate hydrochloride, metoprolol tartrate, minocycline hydrochloride, montelukast sodium, naproxen sodium, neostigmine bromide, nicotinamide, niacin, nifurtimox, nortriptyline hydrochloride, olanzepine, oxybutynin chloride, penicillamine, penicillin V potassium, phenyloin sodium, phenformin, pramipexole, pravastatin sodium, potassium chloride, primaquine phosphate, promethazine, promethazine hydrochloride, proponolol hydrochloride, propoxyphene hydrochloride, pseudophedrine hydrochloride, pyridostigmine bromide, pyridoxine hydrochloride, quinapril hydrochloride, quetiapine, ramipril, ranitidine hydrochloride, reboxetine, risedronate sodium, rosiglitazone maleate, sildenafil, sodium valproate, salbutamol sulfate, stavudine, sumanirole, sumatriptan succinate, terazosin hydrochloride, tetracycline hydrochloride, timolol meleate, tramadol hydrochloride, valacyclovir hydrochloride, vancomycin, venlafaxine hydrochloride, verapamil hydrochloride, warfarin sodium or combinations thereof. Highly water soluble drugs, such as methylphenidate and its salts including methylphenidate hydrochloride, benefit most from the dosage form of the invention. The pharmaceutical agent is present in an amount from about 2 wt.% to about 25 wt.% of the total weight of the uncoated matrix core, preferably from about 4 wt.% to about 16 wt.%, and more preferably from about 5 wt.% to about 11 wt.%.

The hydrophobic material within the matrix core acts as a release rate retarding agent. The hydrophobic material is any non-swellable hydrophobic material comprising at least one polymer. Non-limiting examples of suitable hydrophobic materials include a glyceride (e.g., glyceryl behenate, glyceryl trimyristate, glyceryl trilaurate, glyceryl tristearate, glyceryl monostearate, glyceryl palmitostearate, or glyceryl triacetate), hydrogenated castor oil, a hydrogenated vegetable oil, a water insoluble cellulose (e.g., ethyl cellulose, cellulose acetate, cellulose acylate, cellulose diacylate, cellulose triacylate, cellulose acetate butyrate, cellulose acetate propionate, nitrocellulose, cellulose diacetate, or cellulose triacetate), a wax or a wax-like substance (e.g., carnauba wax, cetyl esters wax, beeswax, castor wax, cationic emulsifying wax, cetrimide emulsifying wax, an emulsifying wax, microcrystalline wax, a nonionic wax, a nonionic emulsifying wax, paraffin, petroleum wax, petroleum ceresin wax, spermaceti wax, white wax, or yellow wax), a fat, an oil, a fatty acid, an emulsifier, a modified starch, a fatty alcohol, a protein (e.g., zein), shellac, or a polymer (e.g., a polyolefin, a polyurethane, a polyvinylchloride, a polyvinyl acetate, an acrylic acid polymer, a methacrylic acid polymer). These and other suitable hydrophobic materials are described in Kibbe, Authur H., Handbook of Pharmaceutical Excipients, 3d Ed. (2000) and Remington's Pharmaceutical Sciences, 18th Ed. (1990). Preferably the hydrophobic material comprises ethyl cellulose, such as AQUALON™ T10 EC, and/or glyceryl behenate, such as COMPRITOL™ 888 ATO. The hydrophobic material is present in an amount from about 10 wt.% to about 50 wt.% of the total weight of the uncoated matrix core, preferably from about 12 wt.% to about 40 wt.%, and more preferably from about 19 wt.% to about 30 wt.%.

The matrix core can also include a release modifier to modify the release rate of the pharmaceutical agent from the matrix core. Exemplary release modifiers include, but are not limited to, hydrophilic celluloses (e.g., hydroxypropyl cellulose, hypromellose, hypromellose phthalate, methyl cellulose, carboxymethyl cellulose sodium, hydroxyethyl cellulose, carboxymethyl hydroxyethyl cellulose, ethyl hydroxyethyl cellulose, ethyl methyl cellulose, hydroxyethyl methyl cellulose, or hydroxymethyl propyl cellulose); saccharides (e.g., pullulan, dextran, sucrose, glucose, fructose, mannitol, lactose, mannose, galactose, or sorbitol); polysaccharides (e.g., polydextrose, guar gum, hydroxypropyl guar, alginate, polysorbate, xanthan gum or carboxymethyl hydroxypropyl guar), polyvinylpyrrolidone, and zein. Preferred release modifiers include high viscosity hydroxypropyl cellulose (KLUCEL™ HXF) and lower viscosity hydroxypropyl cellulose (KLUCEL™ EF). When included, the release modifier is present in an amount up to about 40 wt.%, from about 5 wt.% to about 20 wt.%, from about 3 wt.% to about 17 wt.% of the total weight of the uncoated matrix core, preferably from about 4 wt.% to about 14 wt.%, and more preferably from about 5 wt.% to about 10 wt.%. In some embodiments, the release modifier is present in an amount from about 10 wt.% to about 17 wt.%.

The matrix core of the solid dosage forms of the present invention can include at least one pharmaceutically acceptable filler as an excipient. The term "fillers" used herein means the fillers or binders which are used for ordinary pharmaceutical production, and includes excipients which facilitate the flow and compression of powdery materials and give the solid dosage forms strength. The following are non-limiting examples of suitable fillers for use in the matrix core of the present invention: microcrystalline cellulose, sodium citrate, dicalcium phosphate, colloidal silicon dioxide, starches, lactose, sucrose, glucose, mannitol, and silicic acid, alginates, gelatin, polyvinylpyrrolidinone, lactitol, dextrose, acacia, hypromellose, hydroxypropyl cellulose, hydroxyethyl cellulose, starch, and pregelatinized starch, with microcrystalline cellulose, such as PROSOLV™ HD90, being preferred in some embodiments. The filler can be present in an amount up to about 75% of the total weight of the uncoated matrix core. The content of the filler in the matrix core can be increased or decreased based on various factors such as active agent load, active agent solubility, and desired release profile. When a filler is included, the filler is generally present in an amount from about 50% to about 75%, preferably from about 50 wt.% to about 70 wt.%, and more preferably from about 50 wt.% to about 65 wt.% or even from about 50 wt.% to about 58 wt.%.

The matrix core of the solid dosage form of the present invention can further include at least one pharmaceutically acceptable lubricant as an excipient. The term "lubricant" as used herein includes excipients that reduce friction, heat and wear when applied as a surface coating to moving parts within the equipment used to make the matrices, such as dies and punches. Suitable lubricants include, either individually or in combination, a glyceride (e.g., glyceryl behenate (e.g., Compritol™ 888 ATO), glyceryl trimyristate, glyceryl trilaurate, glyceryl tristearate glyceryl monostearate, glyceryl palmitostearate, glyceryl triacetate); stearic acid and salts thereof, including magnesium, calcium, aluminum, zinc, and sodium stearates; hydrogenated vegetable oils (e.g., Sterotex™); colloidal silica; talc; waxes; boric acid; sodium benzoate; sodium acetate; sodium fumarate; DL-leucine; polyethylene glycol (e.g., Carbowax™ 4000 and Carbowax™ 6000); sodium oleate; sodium lauryl sulfate; and magnesium lauryl sulfate. The lubricant is more preferably selected from the group consisting of stearic acid salts such as calcium stearate and magnesium stearate, stearic acid, sodium stearyl fumarate, solid polyethylene glycols, sodium lauryl sulfate, and mixtures thereof. Magnesium stearate is a particularly preferred lubricant. When present, the amount of lubricant in the matrix core is from about 0.5 wt.% to about 5 wt.% of the total weight of the uncoated matrix core, preferably from about 0.5 wt.% to about 3 wt.%, and more preferably from about 1 wt.% to about 2 wt.%.

The matrix core can also include a glidant to improve flow of the mixture through the hopper used in forming the matrices. Suitable glidants include colloidal silicon dioxide (such as Cab-O-Sil™, M5), aluminum silicate, talc, powdered cellulose, magnesium trisilicate, silicon dioxide, kaolin, glycerol monostearate, metal stearates such as magnesium stearate, titanium dioxide and starch. Colloidal silicon dioxide is a preferred glidant. When present, the amount of glidant in the matrix core is from about 1 wt.% to about 10 wt.% of the total weight of the uncoated matrix core, preferably from about 2 wt.% to about 7 wt.%, and more preferably from about 2 wt.% to about 4 wt.%.

Other excipients for use in the matrix core, such as colorants, flavors, sweeteners and stabilizers, are known in the pharmaceutical art and can be used in compositions of the present invention.

In some embodiments, the matrix core comprises from about 2 to about 25 wt.% of the water soluble pharmaceutical agent, up to about 5 wt.% lubricant, up to about 75 wt.% filler, up to about 25 wt.% release modifier, and up to about 10 wt.% glidant. In some other embodiments, the matrix core comprises from about 12 to about 40 wt.% of the hydrophobic material, from about 4 to about 16 wt.% of the water soluble pharmaceutical agent, from about 0.5 to about 3 wt.% lubricant, from about 50 to about 70 wt.% filler, from about 5 to about 20 wt.% release modifier, and up to about 7 wt.% glidant. In yet other embodiments, the matrix core comprises from about 19 to about 30 wt.% of the hydrophobic material, from about 5 to about 11 wt.% of the water soluble pharmaceutical agent, from about 1 to about 2 wt.% lubricant, from about 50 to about 58 wt.% filler, from about 10 to about 17 wt.% release modifier, and up to about 4 wt.% glidant.

The barrier coating of the solid dosage form comprises a water soluble polymer and optional anti-tacking agents or optional viscosity enhancing agents. The water soluble polymer of the barrier coating can be any water soluble polymer and is preferably a highly water soluble polymer. Suitable materials for use as the barrier coating include hypromellose, hypromellose phthalate, methyl cellulose, carboxymethyl cellulose sodium, hydroxyethyl cellulose, hydroxypropyl cellulose, carboxymethyl hydroxyethyl cellulose, ethylhydroxy ethyl cellulose, ethyl methyl cellulose, hydroxyethyl methyl cellulose, hydroxymethyl cellulose, hydroxymethyl propyl cellulose, polyvinyl alcohol, polyvinylpyrrolidone, a polyalkylene oxide, a polyalkylene glycol, an acrylic acid polymer, a vinyl acetate copolymer, a polysaccharide, a methacrylic acid copolymer, or a maleic anhydride/methyl vinyl ether copolymer. In an embodiment, the barrier coating contains hypromellose. The barrier coating is used in an amount which is a weight gain of from about 2.0 wt.% to about 6.0 wt.% of the total weight of the uncoated matrix core, preferably from about 3 wt.% to about 5 wt.%, and more preferably from about 3.5 wt.% to about 4.5 wt.%.

In some embodiments, the barrier coating may include an anti-tacking agent to reduce agglomeration of the coating during application. Nonlimiting examples of anti-tacking agents for use in the barrier coating include talc, glyceryl monostearate, magnesium stearate, calcium stearate, stearic acid, and mixtures thereof. When present, the amount of anti-tacking agent in the barrier coating is from about 5 wt.% to about 50 wt.% based on the total weight of the barrier coating.

Other excipients for use in the barrier coating, such as viscosity modifiers, are known in the pharmaceutical art and can be used in compositions of the present invention.

The modified release coating includes a hydrophobic polymer, a hydrophilic pore-forming agent, and optional plasticizers and anti-tacking agents. Any hydrophobic polymer can be used in the modified release coating of the solid dosage form. Non-swelling polymers are preferred. The hydrophobic polymers suitable for use in the modified release coating in the present invention include, but are not limited to, ethyl cellulose, cellulose acetate, cellulose acetate butyrate, cellulose acetate propionate, nitrocellulose, polyvinyl chloride, polyvinyl acetate, polymethylmethacrylate, polyethylacrylate, polyacrylates, polymethacrylates such as Eudragit™ (an ammonio methacrylate copolymer available from Rohm Pharma, Darmstadt, Germany), polyvinylchloride, polyethylene, polypropylene, polyisobutylene, polytrimethylammonioethylmethacrylate or a block polymer or copolymer thereof. The hydrophobic polymer is present in an amount from about 5 wt.% to about 70 wt.% of the total weight of the modified release coating, preferably from about 20 wt.% to about 60 wt.%, and more preferably from about 25 wt.% to about 55 wt.%.

Ethyl cellulose is a preferred hydrophobic polymer for use in the modified release coating. Ethyl cellulose can be a standard ethyl cellulose dispersion that contains ethyl cellulose, a suitable plasticizer, and stabilizers. An example of a suitable grade of ethyl cellulose dispersion is available from Colorcon, Inc. of West Point, Pa., under the tradename SURELEASE™, which contains approximately 25% solids by weight.

Any hydrophilic pore-forming agent can be used in the modified release coating and can be a solid or liquid. Suitable pore-forming agents include, but are not limited to, a polymer (e.g., polyvinylpyrrolidone, a polyalkylene oxide, or a polyalkylene glycol), a cellulose or a cellulose ether (e.g., hypromellose, hypromellose phthalate, methyl cellulose, carboxymethyl cellulose sodium, hydroxyethyl cellulose, hydroxypropyl cellulose, carboxymethyl hydroxyethyl cellulose, ethyl hydroxyethyl cellulose, ethyl methyl cellulose, hydroxyethyl methyl cellulose, hydroxymethyl cellulose, or hydroxymethyl propyl cellulose), a protein, a protein derivative, a saccharide (e.g., pullulan, dextran, sucrose, glucose, fructose, mannitol, lactose, mannose, galactose, or sorbitol), a polysaccharide (e.g., polydextrose, guar gum, hydroxypropyl guar, alginate, xanthum gum or carboxymethyl hydroxypropyl guar), an alkali metal salt (e.g., lithium carbonate, sodium chloride, sodium bromide, potassium chloride, potassium sulfate, potassium phosphate, sodium acetate, or sodium citrate). The hydrophilic pore forming agent is present in an amount from about 30 wt.% to about 95 wt.% of the total weight of the modified release coating, preferably from about 40 wt.% to about 80 wt.%, and more preferably from about 45 wt.% to about 75 wt.%.

Preferably, the pore-forming agent is a hydroxyalkyl alkyl cellulose, carboxymethyl cellulose, or salt thereof, such as hydroxyethyl methyl cellulose, hypromellose, carboxymethyl cellulose, or sodium carboxymethyl cellulose. Most preferably, the water-soluble component is hypromellose, such as OPADRY™ (available from Colorcon, West Point, Pa.).

In some embodiments, the modified release coating may include an anti-tacking agent to reduce agglomeration of the coating during application. Nonlimiting examples of anti-tacking agents for use in the modified release coating include talc, glyceryl monostearate, magnesium stearate, calcium stearate, stearic acid, and mixtures thereof. When present, the amount of anti-tacking agent in the modified release coating is from about 5 wt.% to about 50 wt.% based on the total weight of the modified release coating.

In one embodiment, the modified release coating includes SURELEASE™ (available from Colorcon) as the hydrophobic polymer and OPADRY™ (available from Colorcon) as the pore-forming agent. Weight ratios of 70:30, 80:20, 90:10 and 95:5 of SURELEASE™ to OPADRY™ were tested, but coatings with equal to or more than 80% SURELEASE™ showed variable release for some coatings. When the cores are coated with ethyl cellulose and hypromellose in ratios less than 80:20, a desired drug release profile with zero-order characteristics is achieved.

In some instances, the modified release coating contains from about 5 to about 70 wt.% of the hydrophobic polymer, from about 30 to about 95 wt.% of the hydrophilic pore-forming agent, up to about 40 wt.% plasticizer, and up to 50 wt.% anti-tacking agent. In other instances, the modified release coating includes from about 20 to about 60 wt.% of the hydrophobic polymer, from about 40 to about 80 wt.% of the hydrophilic pore-forming agent, from about 20 to about 35 wt.% plasticizer, and up to 20 wt.% anti-tacking agent. In yet other instances, the modified release coating comprises from about 25 to about 55 wt.% of the hydrophobic polymer, from about 45 to about 75 wt.% of the hydrophilic pore-forming agent, and from about 20 to about 30 wt.% plasticizer, and up to 10 wt.% anti-tacking agent.

In one embodiment of the invention, the weight ratio of the hydrophobic polymer to the hydrophilic pore-forming agent is from about 1:1 to about 9:1, about 1:1 to about 4:1, about 1:1 to about 3:1, and preferably from about 1.25:1 to about 2.33:1.

In the results of Example 3, it was discovered that polymer coating of mixtures of hydrophobic ethyl cellulose and hydrophilic hypromellose polymers are susceptible to film cracking. This can result in changes in dissolution profile upon storage. Film cracking can be overcome by imparting flexibility to the modified release coating and/or by adding plasticizer. SURELEASE™ and OPADRY™ both contain some plasticizer. However, an additional amount of at least about 4, 5, 6, 7, 8, 9 or 10 wt.% plasticizer or more is added to prevent the coating from cracking under storage conditions, as shown by the results of Example 3. Any plasticizer for tablet coatings can be used. Exemplary plasticizers are triacetin, methyl abietate, acetyl tributyl citrate, acetyl triethyl citrate, diisooctyl adipate, amyl oleate, butyl ricinoleate, benzyl benzoate, butyl and glycol esters of fatty acids, butyl diglycol carbonate, butyl oleate, butyl stearate, di(β-methoxyethyl) adipate, dibutyl sebacate, dibutyl tartrace, diisobutyl adipate, dihexyl adipate, triethylene glycol di(2-ethyl butyrate), polyethylene glycol di(2-ethyl hexoate), diethylene glycol monolaurate, monomeric polyethylene ester, hydrogenated methyl ester of rosin, methoxyethyl oleate, butoxyethyl stearate, butyl phthalyl butyl glycolate, glycerol tributyrate, triethylene glycol dipelargonate, β-(p-tert-amylphenoxy)ethanol, β-(p-tert.-butylphenoxy)ethanol, β-(p-tert-butylphenoxyethyl)-acetate, bis(β-p-tert-butylphenoxydiethyl)ether, camphor, a petroleum based hydrocarbon, diamyl phthalate, (diamylphenoxy)ethanol, diphenyl oxide, hydrobiethylalcohol, beckolin, acetyltributylcitrate, polyethyleneglycol, blown castor oil, or glyceryl triacetate. In one embodiment the plasticizer was triacetin. When present, the amount of added plasticizer in the release coating is from about 3 wt.% to about 20 wt.% of the total weight of the modified release coating, preferably from about 4 wt.% to about 15 wt.%, and more preferably from about 5 wt.% to about 10 wt.%. When present, the amount of total plasticizer in the release coating is from about 20 wt.% to about 40 wt.% of the total weight of the modified release coating, preferably from about 20 wt.% to about 35 wt.%, and more preferably from about 20 wt.% to about 30 wt.%.

The solid dosage form of the invention can optionally include an overcoating to provide an initial dose burst of an active agent. The overcoating contains any pharmaceutically active agent, a water soluble polymer, an optional anti-tacking agent, an optional plasticizer, an optional stabilizer, and an optional viscosity enhancing agent. The overcoating, if present, is applied to the modified release coated, optionally barrier coated matrix core tablet. Examples of such active agents include any water insoluble drugs, as well as any water soluble drug such as those listed above with regard to the active agent of the matrix core. In some cases, a water soluble active agent is preferred in the overcoating. In some embodiments, the pharmaceutically active agent within the overcoating is the same active agent as included in the matrix core. In other embodiments, the pharmaceutically active agent within the overcoating is different than the active agent as included in the matrix core.

In some embodiments, the active agent is not highly water soluble and forms a suspension rather than a solution with the water soluble polymer. The particle size of the active agent and any other undissolved components in such overcoating suspension can be in the micron range to minimize settling of these components in the suspension and to maintain a homogeneous suspension.

The water soluble polymer of the overcoating can be any water soluble polymer and is preferably a highly water soluble polymer. Suitable materials for use in the overcoating include hypromellose, hypromellose phthalate, methyl cellulose, carboxymethyl cellulose sodium, hydroxyethyl cellulose, hydroxypropyl cellulose, carboxymethyl hydroxyethyl cellulose, ethylhydroxy ethyl cellulose, ethyl methyl cellulose, hydroxyethyl methyl cellulose, hydroxymethyl cellulose, hydroxymethyl propyl cellulose, polyvinyl alcohol, polyvinylpyrrolidone, a polyalkylene oxide, a polyalkylene glycol, an acrylic acid polymer, a vinyl acetate copolymer, a polysaccharide, a methacrylic acid copolymer, or a maleic anhydride/methyl vinyl ether copolymer. In some embodiments, the overcoating contains hypromellose. When present, the overcoating is used in an amount which is a weight gain of from about 2 wt.% to about 25 wt.% of the total weight of the overcoated solid dosage form, preferably from about 4 wt.% to about 12 wt.%, and more preferably from about 5 wt.% to about 6.5 wt.%.

The overcoating may contain a plasticizer. Any plasticizer for tablet coatings can be used. Exemplary plasticizers are those described above for the overcoating. In some embodiments, the plasticizer is triacetin or polyethylene glycol. When present, the amount of plasticizer in the overcoating is from about 5 wt.% to about 30 wt.% of the total weight of the overcoating.

In some embodiments, the overcoating may include an anti-tacking agent to reduce agglomeration of the coating during application. Nonlimiting examples of anti-tacking agents for use in the overcoating include talc, glyceryl monostearate, magnesium stearate, calcium stearate, stearic acid, and mixtures thereof. When present, the amount of anti-tacking agent in the overcoating is from about 5 wt.% to about 50 wt.% based on the total weight of the overcoating.

The overcoating can include an optional stabilizer to minimize or prevent degradation of the pharmaceutically active agent in the overcoating. Any pharmaceutically acceptable compound which minimizes degradation of the pharmaceutically active agent in the overcoating can be used. For example, an acidic compound such as hydrochloric acid or fumaric acid can be used to stabilize the active agent when the active agent is more stable in acidic pH. For example, an acid can be used to stabilize methylphenidate. One of ordinary skill in the art could readily identify suitable stabilizers for the particular active agents of the dosage forms of the invention. When present, the amount of stabilizer in the overcoating is from about 1 wt.% to about 10 wt.% based on the total weight of the overcoating.

The overcoating may also include an optional viscosity enhancing agent to reduce settling of the active agent in the overcoat, particularly when the overcoating is applied as a suspension rather than as a solution. Nonlimiting examples of viscosity enhancing agents for use in the overcoating include a sugar (dextrose, glucose and sucrose), a cellulose derivative such as sodium or calcium carboxymethylcellulose and hydroxypropyl cellulose, a polysaccharide, a pectin, agar, carrageenan, a hydrophilic gum such as acacia gum, guar gum, arabic gum and xanthan gum, alginic acid, an alginate, dextran, a carbomer resin, and mixtures thereof. When present, the amount of viscosity enhancing agent in the overcoating is from about 2 wt.% to about 20 wt.% based on the total weight of the overcoating.

When the solid dosage form is overcoated as described herein, the drug release rate is no longer zero order when the same drug within the matrix core is used in the overcoating. When a different drug is used in the matrix core than is used in the overcoating, the drug release rate of the drug within the matrix core is zero order, but the drug release rate of the drug in the overcoating is not zero order.

In some instances, the overcoating contains from about 25 wt.% to about 77 wt.% of the pharmaceutical agent, from about 23 wt.% to about 75 wt.% of the water soluble polymer, up to about 50 wt.% of the anti-tacking agent, up to about 10 wt.% of the stabilizer, and up to about 20 wt.% of the viscosity enhancing agent. In other instances, the overcoating includes from about 36 wt.% to about 45 wt.% of the pharmaceutical agent, from about 55 wt.% to about 64 wt.% of the water soluble polymer, up to about 5 wt.% of the anti-tacking agent, up to about 1 wt.% of the stabilizer, and up to about 2 wt.% of the viscosity enhancing agent.

In some embodiments, the weight ratio of the water soluble polymer to the pharmaceutical agent in the overcoating is from about 0.3:1 to about 3.0:1, preferably from about 1.2:1 to about 1.8:1.

The matrix core, overcoating, modified release coating and/or barrier coating can include additional conventional excipients known in the art. Through selection and combination of excipients, solid dosage forms can be provided exhibiting improved performance with respect to, among other properties, efficacy, bioavailability, clearance time, stability, safety, dissolution profile, disintegration profile and/or other pharmacokinetic, chemical and/or physical properties. Where the composition is formulated as a tablet, the combination of excipients selected provides tablets that can exhibit improvement, among other properties, in dissolution profile, hardness, crushing strength, and/or friability.

The solid dosage forms can be formulated into a variety of physical structures or forms, including tablets, lozenges and caplets. No specialized geometry of the matrix core is necessary in the present invention. The matrix core may be in any shape known in the pharmaceutical industry and suitable for drug delivery, such as in spherical, cylindrical, or conical shape. Tablets are preferred.

The methylphenidate formulations of the present invention may be administered to children and adults and preferably have a duration of action of about 6 hours and not more than about 10 hours. The inventive methylphenidate formulation should be taken at breakfast time and is designed to eliminate the need for dosing at school or work during lunch time. If a patient requires more than 10 hours of treatment per day an additional dose of immediate release methylphenidate should be taken at dinner. If the patient requires 24 hour treatment an additional does of the inventive formulation should be given at dinner time.

The solid dosage forms of the present invention are made by a simple method under ambient conditions without the use of expensive manufacturing equipment. Other methods, such as wet granulation, are more expensive, time consuming, involve more excipients, and require more capital equipment. The method provides ease of manufacture and drug release in a substantially linear fashion over an extended period of time. The method comprises mixing between about 10% and about 50% of the non-swellable hydrophobic material comprising at least one polymer, from about 2 to 25 wt.% of the water-soluble pharmaceutical agent, the optional filler, the optional release modifier, the optional lubricant, and the optional glidant to form a mixture, and compressing the mixture. In one embodiment, the mixture comprises not more than about 9, 10, 11, 12 or 13 wt.% of the water soluble pharmaceutical agent. The mixture can be formed and/or compressed under ambient conditions. Any conventional method of direct compression is suitable for preparing the matrix core.

To include a barrier coating in the dosage form, the matrix core is coated with a water soluble polymer, optionally mixed with an anti-tacking agent, to form a barrier coating surrounding the matrix core. Coating methods are well known in the art. After the barrier coating has dried, it is coated with the modified release coating. Once the modified release coating has dried, the dosage form can be stored. The solid dosage form is capable of releasing the pharmaceutical agent at a zero order rate for a period of at least 4, 5, 6, 7, 8, 9 or 10 hours after administration to a subject.

If the dosage form is to include an overcoating, the modified release coated matrix core is coated with a mixture of the water soluble pharmaceutically active agent, the water soluble polymer, the optional anti-tacking agent, the optional stabilizer, and the optional viscosity enhancing agent, and dried. Conventional coating methods can be used. The overcoated dosage form can be stored.

In one embodiment, the pharmaceutical active agent is placed in a V-blender or like mixing apparatus with the hydrophobic material, optional filler, optional release modifier, optional lubricant, and optional glidant and mixed for several minutes under ambient conditions. The mixture is compressed using a Korsch PH 106 tablet press or other standard tabletting equipment under ambient conditions without use of heat or solvent to form the matrix core. The matrix core is then coated with the barrier coating and the modified release coating using a Vector Hi-coater or comparable coating apparatus. For example, methylphenidate hydrochloride is placed in a V-blender with ethyl cellulose and silicified microcrystalline cellulose and mixed for five minutes. Thereafter, colloidal silicon dioxide is added as a glidant and the mixture is mixed for another five minutes. The mixture is compressed using a Korsch PH 106 tablet press under ambient conditions. The resulting matrix core is then coated with a hypromellose coating and a modified release coating containing hypromellose, ethyl cellulose, and optional triacetin using a Vector Hi-coater.

### Examples

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice.

### Reference Example 1

Methylphenidate hydrochloride was placed in a V-blender with ethyl cellulose, hydroxypropyl cellulose and silicified microcrystalline cellulose and mixed for five minutes. Thereafter, magnesium stearate was added as a lubricant and the mixture was mixed for another five minutes. Then, the mixture was compressed using a Korsch PH 106 tablet press under ambient conditions to form a matrix core. The matrix core contained the ingredients in the table below.

| **Ingredient** | Weight (mg) |
|---|---|
| Methylphenidate HCl | 21 |
| Hydroxypropyl Cellulose (KLUCEL™ HXF) | 25 |
| Ethyl cellulose (AQUALON™ T10 EC) | 75 |
| Silicified Microcrystalline Cellulose (PROSOLV™ HD90) | 126.5 |
| Magnesium Stearate | 2.5 |
| Total | 250 |

The methylphenidate hydrochloride was the water soluble pharmaceutical agent, ethyl cellulose was the hydrophobic material, high viscosity hydroxypropyl cellulose was the release modifier, silicified microcrystalline cellulose was a filler, and magnesium stearate was used as a lubricant.

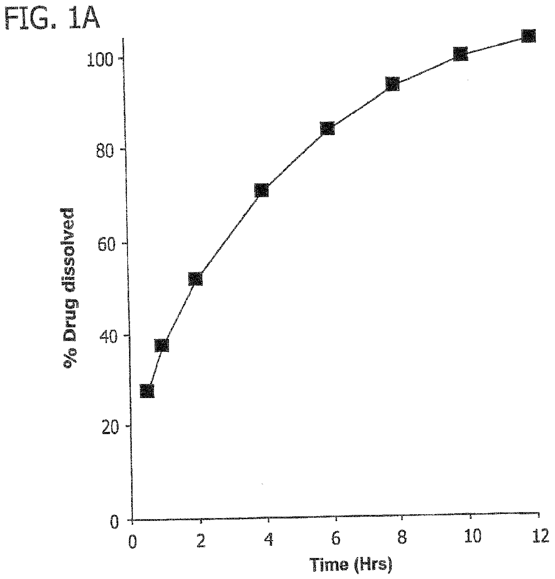

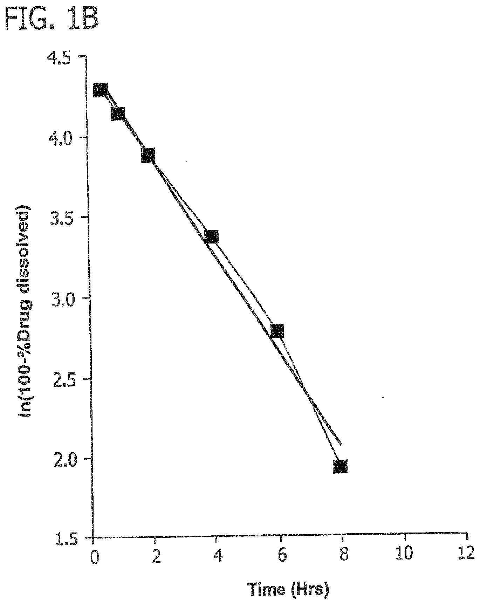

In one example (provided for reference purposes only), the tablets were not coated. The release characteristics of the tablets were tested by placing tablets in 0.1 % formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 1A and were determined via UV spectroscopy at 200 nm. Figure 1B is a plot of ln (100-%methylphenidate hydrochloride dissolved) as a function of time and indicates first order drug release for the uncoated matrix core tablets.

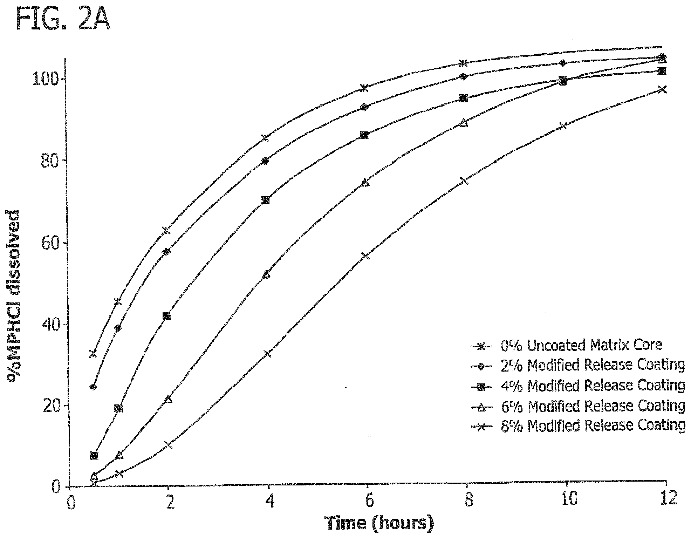

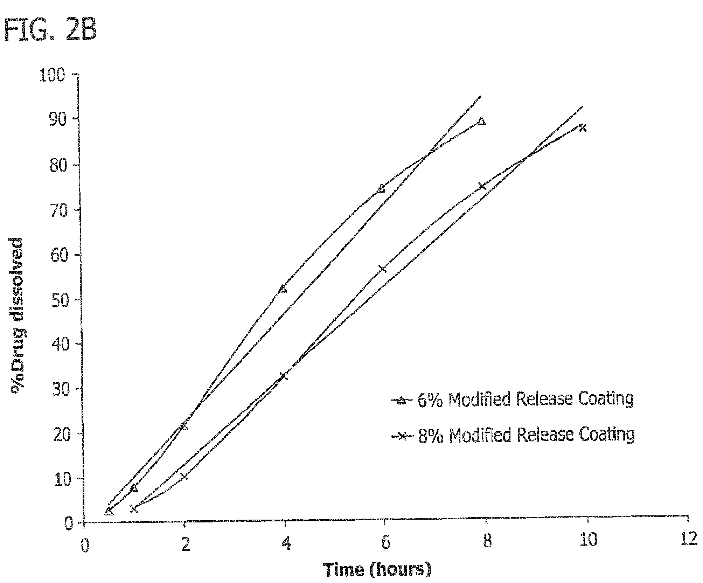

In another reference example, the matrix cores were coated with a modified release coating comprising 70% SURELEASE™ and 30% OPADRY™ (% w/w solid content). The tablets were coated with varying quantities of coating, up to 8 wt.% based on weight gain of the tablet, and some tablets were not coated. The preferred amount of coating to achieve a zero-order release profile was experimentally determined by placing tablets in 0.1% formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 2A, and were determined via UV spectroscopy at 200 nm. Figure 2B is a plot of ln (100-%methylphenidate hydrochloride dissolved) as a function of time and indicates zero order drug release for the coated tablets.

### Reference Example 2

A matrix core was produced according to the method of production of reference example 1. The matrix core contained the ingredients in the table below.

| **Ingredient** | Weight (mg) |
|---|---|
| Methylphenidate HCl | 21 |
| Glyceryl Behenate (COMPRITOL™ 888 ATO) | 31.25 |
| Hydroxypropyl Cellulose (KLUCEL™ EF) | 12.25 |
| Silicified microcrystalline Cellulose (PROSOLY™ HD90) | 185.25 |
| Colloidal Silicon Dioxide | 5.0 |
| Total | 255 |

The methylphenidate hydrochloride was the water soluble pharmaceutical agent, low viscosity hydroxypropyl cellulose was used as a release modifier, glyceryl behenate was added as both the hydrophilic material and as a lubricant, silicified microcrystalline cellulose was a filler, and colloidal silicon dioxide was used as a glidant.

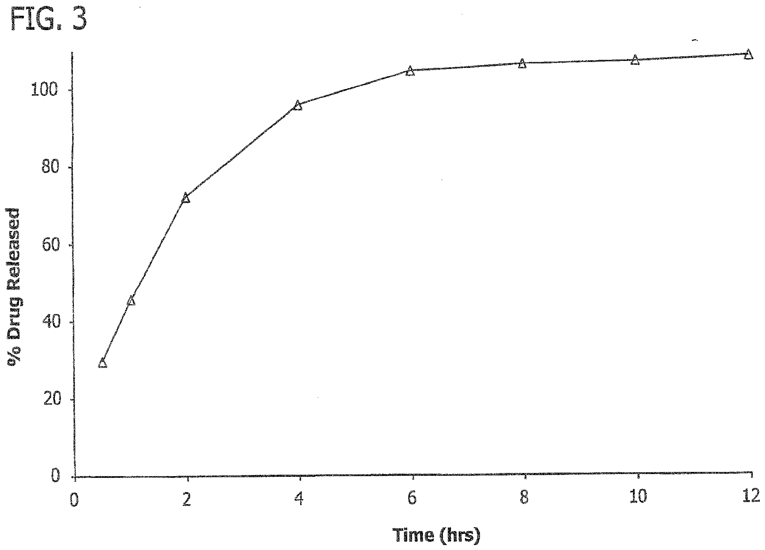

In one example, the tablets were not coated. The release characteristics of the tablets were tested by placing tablets in 0.1 % formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 3, and were determined via UV spectroscopy at 200 nm.

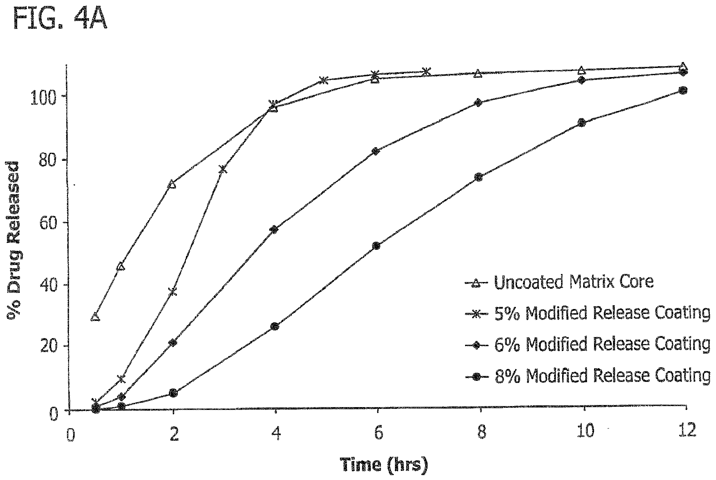

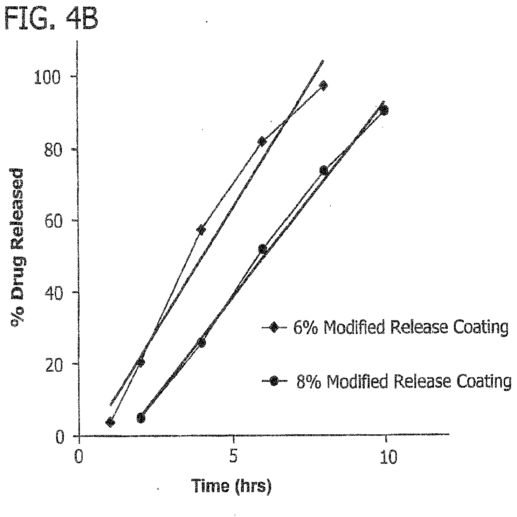

In another example, the matrix cores were coated with a coating comprising 70% SURELEASE™ and 30% OPADRY™ (% w/w solid content). The tablets were coated with varying quantities of coating, up to 8 wt.% based on weight gain of the tablet, and some tablets were not coated. The preferred amount of coating to achieve a zero-order release profile was experimentally determined by placing tablets in 0.1 % formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 4A, and were determined via UV spectroscopy at 200 nm.. Figure 4B is a plot of ln (100-%methylphenidate hydrochloride dissolved) as a function of time and indicates zero order drug release for the 6% and 8% coated tablets.

### Reference Example 3

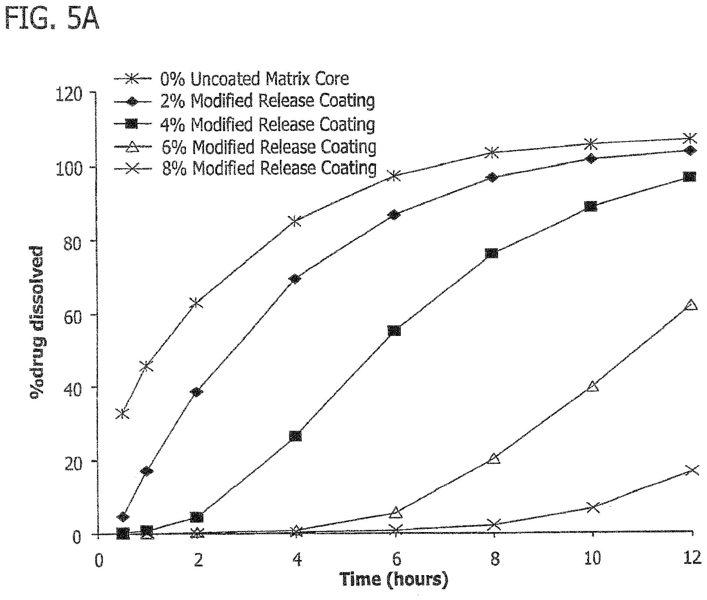

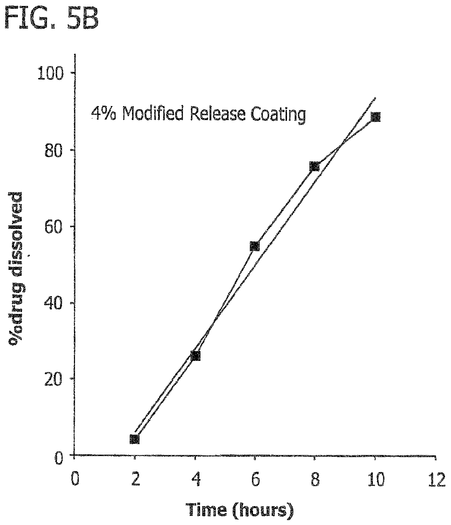

After the matrix cores of reference example 1 were formed, they were coated with a coating comprising 80% SURELEASE™ and 20% OPADRY™ (% w/w solid content). The tablets were coated with varying quantities of coating, up to 8 wt.% based on weight gain of the tablet, and some tablets were not coated. The preferred amount of coating to achieve a zero-order release profile was experimentally determined by placing tablets in 0.1% formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 5A, and were determined via UV spectroscopy at 200 nm. Figure 5B is a plot of ln (100-%methylphenidate hydrochloride dissolved) as a function of time and indicates zero order drug release for the 4% coated tablets. It is expected that the tablets having a 6% or 8% polymer coating would have exhibited a zero-order release profile if the testing were extended beyond 12 hours.

### Reference Example 4

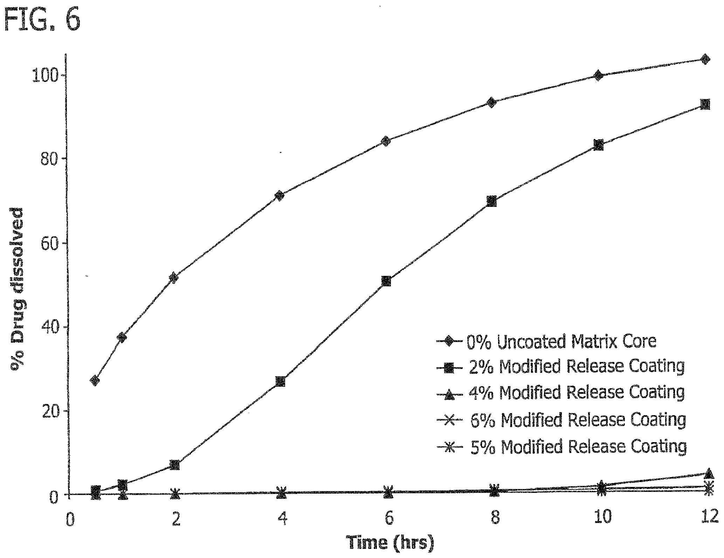

After the matrix cores of reference example 1 were formed, they were coated with a coating comprising 95% SURELEASE™ and 5% OPADRY™ (% w/w solid content). The tablets were coated with varying quantities of coating, up to 6 wt.% based on weight gain of the tablet, and some tablets were not coated. The preferred amount of coating to achieve a zero-order release profile was experimentally determined by placing tablets in 0.1% formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figure 6, and were determined via UV spectroscopy at 200 nm. It is expected that the tablets having a 4, 5 or 6% polymer coating would have exhibited a zero-order release profile if the testing were extended beyond 12 hours.

### Reference Example 5

Matrix core tablets were prepared as in reference Example 1. These were coated with a mixture of ethyl cellulose and hypromellose in ratio of 70:30 w/w. The tablets were placed in an oven at a temperature of 50°C for up to 7 days. The dissolution profiles of tablets before and after oven-heating were compared.

The uncoated matrix tablets provided a dissolution profile with first-order release kinetics. When these matrix tablets were coated to a total weight gain of at least 6 wt.%, a slower drug dissolution rate with zero-order characteristics was observed. However, when the coated tablets were placed in an oven at 50°C, the dissolution rate increased and the shape of the profile reverted to first-order kinetics. This was attributable to the observed rupture of the polymer coating near the edges of the central band of the tablets. As a result of coating failure, the shape of drug release profile of coated tablets assumed that of core matrices. The breaking of the polymer film can be a result of one or more of the following: (i) brittleness of the hybrid polymer film; (ii) expansion of tablet core during oven-heating and/or *in-vitro* dissolution; and (iii) non-uniformity of polymer layer thickness due to preferential coating on the top and bottom faces of the tablet surface. This effect of on the dissolution profile was eliminated by optimizing the level of plasticizer, which increased the film flexibility. Coating thickness uniformity was improved by proper design of tablet tooling.

### Example 1

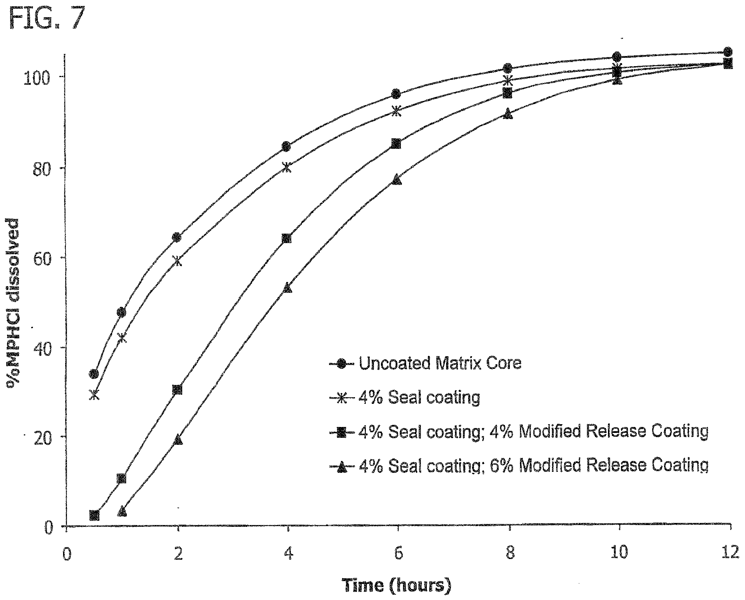

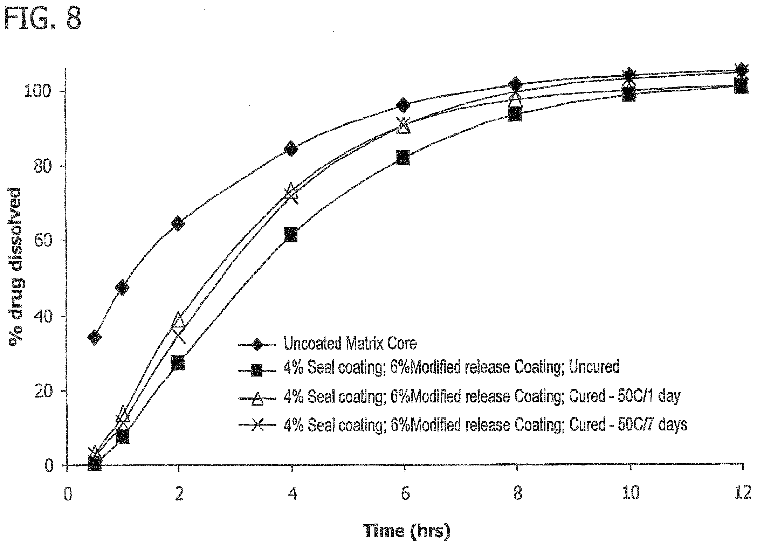

Another means of preventing such cracking is by applying a barrier coating prior to the application of the modified release coating. The same coating apparatus that was used in the prior examples was used to apply the barrier coating and then the modified release coating. The tablets were coated with 4 wt.% barrier coating, based on the weight gain of the matrix core, and either 4 wt.% or 6 wt.% of modified release coating, based on weight gain of the tablet, and some tablets were not coated. In Figure, 8, the 6% modified release coated tablets were either uncured (i.e., no 50°C heating), cured at 50°C for 1 day, or cured at 50°C for 7 days. The preferred amount of coating to achieve a zero-order release profile was experimentally determined by placing tablets in 0.1 % formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figures 7 and 8, and were determined via UV spectroscopy at 200 nm. Visual observation revealed that the coating remained intact during the entire dissolution process.

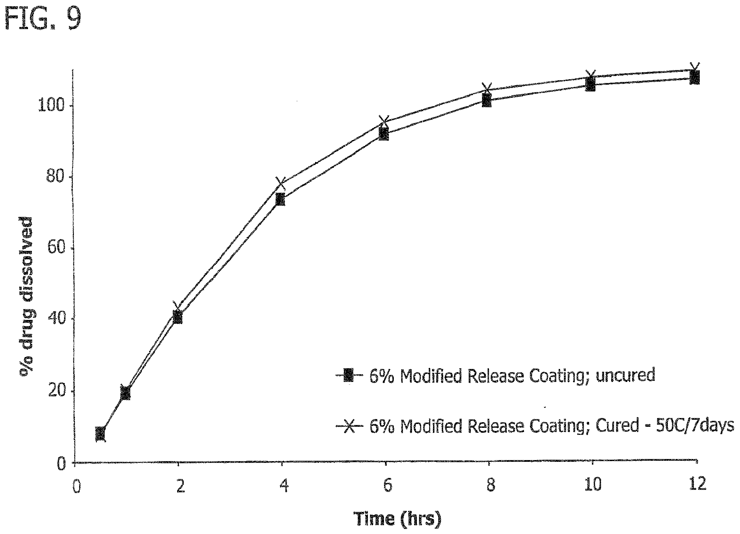

In another embodiment, tablets having a matrix core including ethyl cellulose and hypromellose in a ratio of 1:3, a modified release coating and a barrier coating were prepared as described above, except that the modified release 6% coating included ethyl cellulose, triacetin, and hypromellose in a ratio of 70:3.5:26.5. The results are depicted in Figure 9. The addition of a plasticizer to the modified release coating increased the flexibility of the coating on the tablets cured at 50°C.

### Reference Example 5

A matrix core was produced according to the method of production of reference example 1. The matrix core contained the ingredients in the table below.

| **Ingredient** | **Formula A Weight (mg)** | **Formula B Weight (mg)** |
|---|---|---|
| Methylphenidate HCl | 27 | 27 |
| Glyceryl Behenate (COMPRITOL™ 888 ATO) | 0 | 75 |
| Ethyl Cellulose (AQUALON™ T10 EC) | 135 | 0 |
| Hydroxypropyl Cellulose (KLUCEL™ HXF) | 45 | 0 |
| Hydroxypropyl Cellulose (KLUCEL™ EF) | 0 | 15 |
| Silicified Microcrystalline Cellulose (PROSOLV™ HD90) | 90 | 183 |
| Magnesium Stearate | 3 | 0 |
| Total | 300 | 300 |

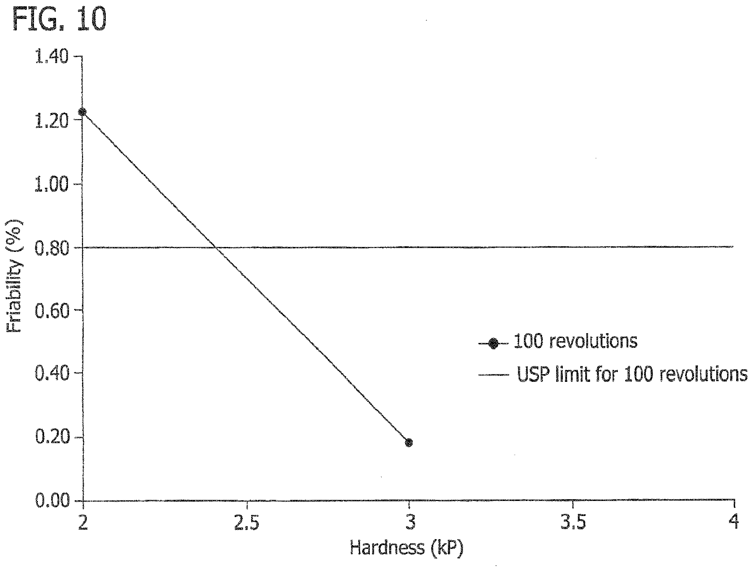

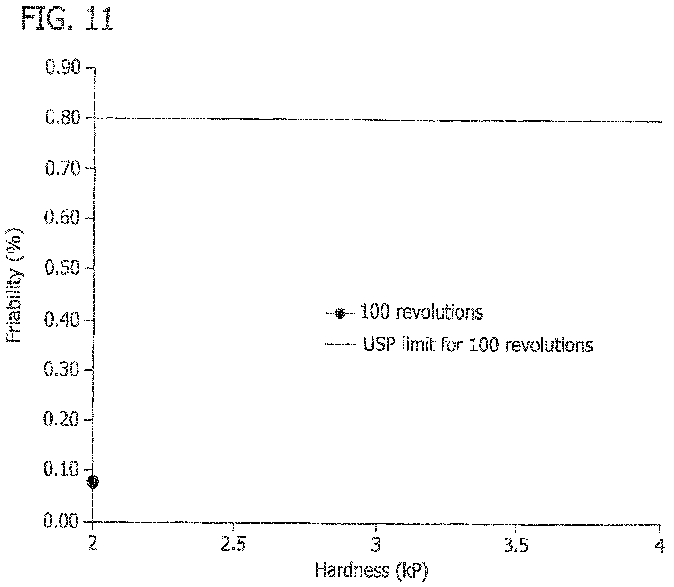

Friability of the uncoated tablets was tested using a USP friability tester at 100 revolutions at different hardness values for both formulations. The results are shown in Figures 10 and 11, wherein "kP" indicates kilo pounds, a unit of force for expressing hardness or crushing strength of pharmaceutical tablets, and "kN" indicates kilo Newtons, a unit of compression force.

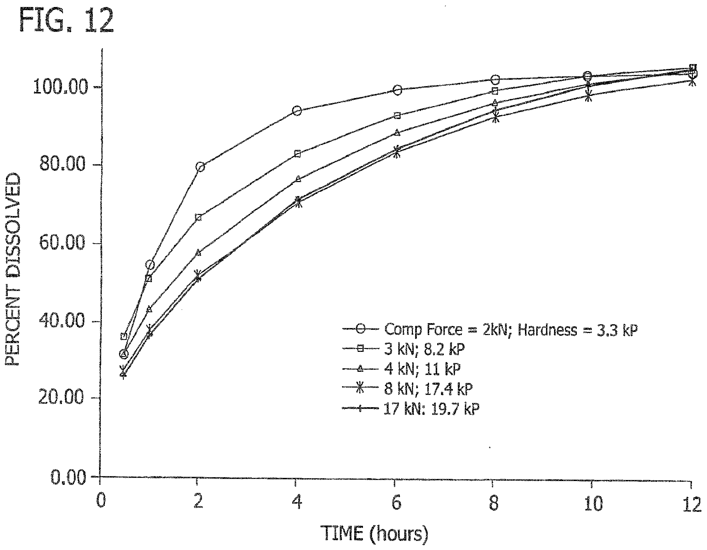

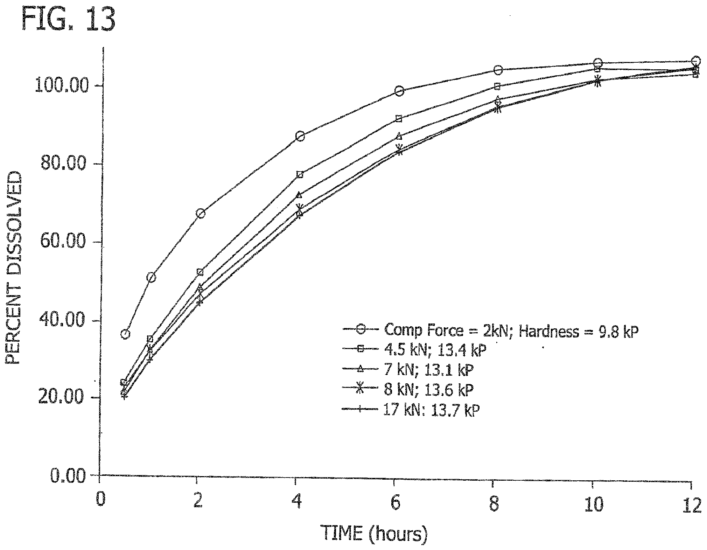

Uncoated matrix core tablets prepared at various hardness and under various compression forces were also placed in 0.1% formic acid aqueous solution (pH 2.6). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figures 12 and 13, and were determined via UV spectroscopy at 200 nm.

### Reference Example 6

Uncoated matrix cores were prepared as in reference example 1 to contain the following ingredients. Upon dissolution, the matrix cores showed similar dissolution to that reported for other matrix cores of the invention.

| **Ingredient** | **Formula I Weight (mg)** | **Formula II Weight (mg)** |
|---|---|---|
| Methylphenidate HCl | 14 | 28 |
| Ethyl Cellulose (AQUALON™ T10 EC) | 75 | 75 |
| Hydroxypropyl Cellulose (KLUCEL™ HXF) | 25 | 25 |
| Hydroxypropyl Cellulose (KLUCEL™ EF) | 0 | 40 |
| Silicified Microcrystalline Cellulose (PROSOLV™ HD90) | 133.5 | 228 |
| Magnesium Stearate | 2.5 | 4 |
| Total | 250 | 400 |

### Example 2

Matrix core tablets including a barrier coating were prepared and then coated with a modified release coating as described in the examples and reference examples above. These tablets were then overcoated with an overcoat solution and dried. The overcoated tablets were further coated with a colorant coating and dried to form the final tablet. The formulations were prepared to contain the ingredients as shown below:

| **Ingredient** | **Formula C Weight (mg)** | **Formula D Weight (mg)** |
|---|---|---|
| **Matrix Core** | | |
| Methylphenidate HCl | 21 | 21 |
| Hydroxypropyl Cellulose (KLUCEL™ HXF) | 25 | 25 |
| Ethyl Cellulose (AQUALON™ T10 EC) | 75 | 75 |
| Silicified Microcrystalline Cellulose (PROSOLV™ HD90) | 126.5 | 126.5 |
| Magnesium Stearate | 2.5 | 2.5 |

| **Barrier Coating** | | |
|---|---|---|
| OPADRY™ CLEAR | 10.4 | 10.4 |

| **Modified Release Coating** | | |
|---|---|---|
| SURELEASE™ CLEAR | 11.6 | 20.3 |
| Triacetin | 0.6 | 1.0 |
| OPADRY™ CLEAR | 4.4 | 7.7 |

| Overcoating | | |
|---|---|---|
| Methylphenidate HCl | 6 | 6 |
| OPADRY™ CLEAR | 9.8 | 10.5 |

| **Color coating** | | |
|---|---|---|
| OPADRY™ II GRAY | 9.1 | 9.5 |
| Total | 301.9 | 315.4 |

| **Ingredient** | **Formula E Weight (mg)** | **Formula F Weight (mg)** |
|---|---|---|
| **Matrix Core** | | |
| Methylphenidate HCl | 42 | 42 |
| Hydroxypropyl Cellulose (KLUCEL™ HXF) | 25 | 25 |
| Ethyl Cellulose (AQUALON™ T10 EC) | 75 | 75 |
| Hydroxypropyl Cellulose (KLUCEL™ EF) | 40 | 40 |
| Silicified Microcrystalline Cellulose (PROSOLV™ HD90) | 214 | 214 |
| Magnesium Stearate | 4 | 4 |

| **Barrier Coating** | | |
|---|---|---|
| OPADRY™ CLEAR | 16.7 | 16.7 |

| **Modified Release Coating** | | |
|---|---|---|
| SURELEASE™ CLEAR | 16 | 34.1 |
| Triacetin | 0.9 | 2.0 |
| OPADRY™ CLEAR | 9.7 | 20.7 |

| **Overcoating** | | |
|---|---|---|
| Methylphenidate HCl | 12 | 12 |
| OPADRY™ CLEAR | 16.3 | 17.6 |

| **Color coating** | | |
|---|---|---|
| OPADRY™ II GRAY | 14.6 | 15.6 |
| Total | 486.2 | 518.7 |

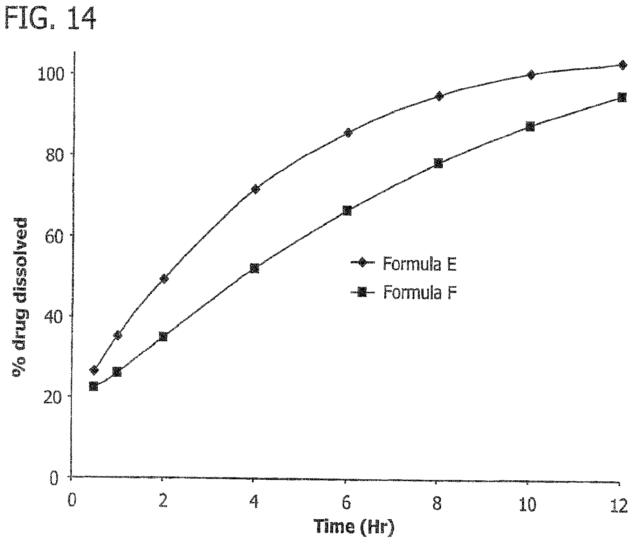

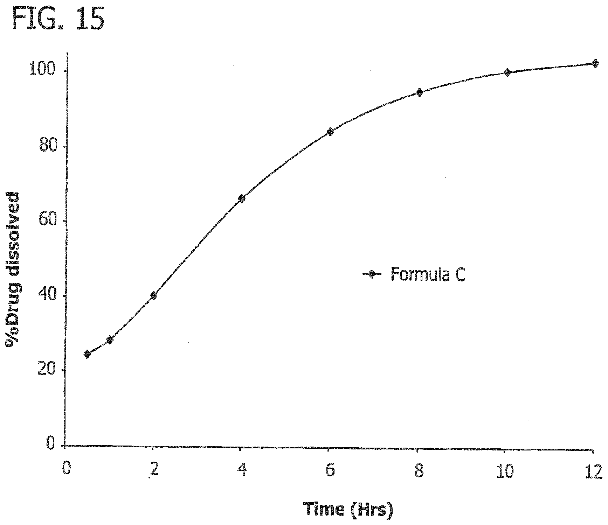

The tablets of Formulae E and F and of Formula C were placed in 0.1% hydrochloric acid aqueous solution (pH 3.0). USP paddle apparatus was used at 50 rpm to mix the tablets in the acid solution. A total volume of 500 ml of the dissolution media was used. At 30 minutes, 1 hour, 2 hours, 4 hours, 6 hours, 8 hours, 10 hours, and 12 hours, the amount of methylphenidate hydrochloride dissolved in each trial run was determined. The experimental results are depicted in Figures 14 and 15, respectively, and were determined via UV spectroscopy at 200 nm.

When introducing elements of the present invention or the preferred embodiment(s) thereof, the articles "a," "an," "the," and "said" are intended to mean that there are one or more of the elements. The terms "comprising," "including," and "having" are intended to be inclusive and mean that there may be additional elements other than the listed elements.

In view of the above, it will be seen that the several objects of the invention are achieved and other advantageous results attained.

## Claims

1. A solid dosage form comprising:
a matrix core which comprises between about 10% and about 50% of a non-swellable hydrophobic material comprising at least one polymer and a water-soluble pharmaceutical agent; and
a modified release coating surrounding the matrix core, wherein the modified release coating comprises a hydrophobic polymer and a hydrophilic pore-forming agent;
a water-soluble barrier coating between the modified release coating and the matrix core such that the barrier coating surrounds the matrix core and the modified release coating surrounds the barrier coating;
wherein the solid dosage form is capable of releasing the pharmaceutical agent at a zero order release rate for a period of at least four hours after administration to a subject.

2. The dosage form of claim 1 wherein the solid dosage form is capable of releasing the pharmaceutical agent at a zero order release rate for from about six to about ten hours after administration to a subject.

3. The dosage form of any one of the preceding claims wherein the water-soluble pharmaceutical agent comprises abacavir sulfate, acyclovir, aminocaproic acid, alendronate sodium, amitriptyline hydrochloride, amphetamine, acetaminophen, allopurinol, amoxicillin, atenolol, atropine sulfate, azithromycin, balsalazide, benzepril hydrochloride, bisoprolol fumarate, bupropion hydrochloride, buformin, calacyclovir, captopril, cefprozil, cetrizine hydrochloride, cimetidine, ciprofloxacin, clindamycin, chlorpheniramine maleate, chlorpromazine hydrochloride, clomipramine hydrochloride, clonidine hydrochloride, clopidogrel bisulfate, cloxacillin sodium, codeine phosphate, colchicines, cyclophosphamide, diethylcarbamazine citrate, diltiazem, doxycycline, doxepin, DL-methionine, eprosartan, ethembutol hydrochloride, ethosuximide, erythromycin, fexofenadine, ferrous sulfate, fluoxetine hydrochloride, fluvastatin, fosonopril sodium, gabapentin, hydralazine hydrochloride, hydrocodone bitartrate, hydroxyzine hydrochloride, hydroxyurea, indinavir sulfate, isoniazid, isosorbide mononitrate, lactobionate, lamivudine, levamisole hydrochloride, levofloxacin, lisinopril, losartan potassium, metformin hydrochloride, methylphenidate, methylphenidate hydrochloride, metoprolol tartrate, minocycline hydrochloride, montelukast sodium, naproxen sodium, neostigmine bromide, nicotinamide, niacin, nifurtimox, nortriptyline hydrochloride, olanzepine, oxybutynin chloride, penicillamine, penicillin V potassium, phenytoin sodium, phenformin, pramipexole, pravastatin sodium, potassium chloride, primaquine phosphate, promethazine, promethazine hydrochloride, propranolol hydrochloride, propoxyphene hydrochloride, pseudophedrine hydrochloride, pyridostigmine bromide, pyridoxine hydrochloride, quinapril hydrochloride, quetiapine, ramipril, ranitidine hydrochloride, reboxetine, risedronate sodium, rosiglitazone maleate, sildenafil, sodium valproate, salbutamol sulfate, stavudine, sumanirole, sumatriptan succinate, terazosin hydrochloride, tetracycline hydrochloride, timolol maleate, tramadol hydrochloride, valacyclovir hydrochloride, vancomycin, venlafaxine hydrochloride, verapamil hydrochloride, warfarin sodium or combinations thereof.

4. The dosage form of any one of the preceding claims wherein the matrix core comprises from about 2 to about 25 wt.% of the water-soluble pharmaceutical agent, up to about 5 wt.% lubricant, up to about 75 wt.% filler, up to about 25 wt.% release modifier, and up to about 10 wt.% glidant.

5. The dosage form of any one of the preceding claims wherein the modified release coating further comprises a plasticizer.

6. The dosage form of any one of the preceding claims wherein the modified release coating comprises from about 5 to about 70 wt.% of the hydrophobic polymer, from about 30 to about 95 wt.% of the hydrophilic pore-forming agent, up to about 40 wt.% plasticizer, and up to 50 wt.% anti-tacking agent.

7. The dosage form of any one of the preceding claims wherein the weight ratio of the hydrophobic polymer to the hydrophilic pore-forming agent is from about 1:1 to about 9:1.

8. The dosage form of any one of the preceding claims wherein the barrier coating comprises a water-soluble polymer.

9. The dosage form of claim 8 wherein the water-soluble polymer comprises hypromellose, hypromellose phthalate, methyl cellulose, carboxymethyl cellulose sodium, hydroxyethyl cellulose, hydroxypropyl cellulose, carboxymethyl hydroxyethyl cellulose, ethylhydroxyethylcellulose, ethylmethylcellulose, hydroxyethyl methylcellulose, hydroxymethyl cellulose, hydroxymethyl propylcellulose, polyvinyl alcohol, polyvinylpyrrolidone, a polyalkylene oxide, a polyalkylene glycol, an acrylic acid polymer, a vinyl acetate copolymer, a polysaccharide, a methacrylic acid copolymer, or a maleic anhydride/methyl vinyl ether copolymer.

10. The dosage form of any one of the preceding claims wherein the barrier coating is present at a weight gain from about 2 to about 6 wt.% based on total weight of the matrix core.

11. The dosage form of any one of the preceding claims further comprising an overcoating surrounding the modified release coating, wherein the overcoating comprises a pharmaceutical agent and a water-soluble polymer.

12. The dosage form of any one of the preceding claims in the form of a tablet.

13. A method of making a solid dosage form comprising:
mixing between about 10% and about 50% of a non-swellable hydrophobic material comprising at least one polymer, from about 2 to 25 wt.% of a water-soluble pharmaceutical agent, an optional filler, an optional lubricant, and an optional glidant to form a mixture;
compressing the mixture to form a matrix core;
coating the matrix core to form a barrier coating surrounding the matrix core;
drying the barrier coating;
coating the barrier-coated matrix core with a modified release coating; and
drying the modified release coating to form the solid dosage form, wherein the barrier coating comprises a water-soluble polymer, the modified release coating comprises a hydrophobic polymer and a hydrophilic pore-forming agent, and the solid dosage form is capable of releasing the pharmaceutical agent at a zero order rate for a period of at least four hours after administration to a subject.

14. The method of claim 13 wherein the method further comprises the steps of coating the solid dosage form with an overcoating, wherein the overcoating comprises a water-soluble polymer and a pharmaceutical agent.

15. The method of claim 13 or 14 wherein the solid dosage form of any one of claims 1-12 is made.

## Patentansprüche

1. Eine feste Dosierform, umfassend:
Einen Matrixkern, welcher zwischen etwa 10% und etwa 50% eines nicht quellbaren hydrophoben Materials enthält, enthaltend wenigstens ein Polymer und ein wasserlösliches pharmazeutisches Mittel; und
eine modifizierte Freisetzungsbeschichtung, welche den Matrixkern umgibt, wobei die modifizierte Freisetzungsbeschichtung ein hydrophobes Polymer und ein hydrophiles porenbildendes Mittel enthält;
eine wasserlösliche Barrierebeschichtung zwischen der modifizierten Freisetzungsbeschichtung und dem Matrixkern, so dass die Barrierebeschichtung den Matrixkern umgibt und die modifizierte Freisetzungsbeschichtung die Barrierebeschichtung umgibt;
wobei die feste Dosierform in der Lage ist, das pharmazeutische Mittel mit einer Freisetzungsgeschwindigkeit nullter Ordnung während einer Dauer von wenigstens vier Stunden nach Verabreichung an einen Probanden freizusetzen.

2. Die Dosierform nach Anspruch 1, wobei die feste Dosierform in der Lage ist, das pharmazeutische Mittel mit einer Freisetzungsgeschwindigkeit nullter Ordnung innerhalb von etwa sechs bis etwa zehn Stunden nach Verabreichung an einen Probanden freizusetzen.

3. Die feste Dosierform nach irgendeinem der vorhergehenden Ansprüche, wobei das wasserlösliche pharmazeutische Mittel umfasst: Abacavirsulfat, Acyclovir, Aminocapronsäure, Alendronat-Natrium, Amitriptylinhydrochlorid, Amphetamin, Acetaminophen, Allopurinol, Amoxicillin, Atenolol, Atropinsulfat, Azithromycin, Balsalazid, Benzeprilhydrochlorid, Bisoprololfumarat, Bupropionhydrochlorid, Buformin, Calacyclovir, Captopril, Cefprozil, Cetrizinhydrochlorid, Cimetidin, Ciprofloxacin, Clindamycin, Chlorpheniraminmaleat, Chlorpromazinhydrochlorid, Clomipraminhydrochlorid, Clonidinhydrochlorid, Clopidogrelbisulfat, Cloxacillin-Natrium, Codeinphosphat, Colchicin, Cyclophosphamid, Diethylcarbamazincitrat, Diltiazem, Doxycyclin, Doxepin, DL-Methionin, Eprosartan, Ethembutolhydrochlorid, Ethosuximid, Erythromycin, Fexofenadin, Eisensulfat, Fluoxetinhydrochlorid, Fluvastatin, Fosonopril-Natrium, Gabapentin, Hydralazinhydrochlorid, Hydrocodonbitartrat, Hydroxyzinhydrochlorid, Hydroxyharnstoff, Indinavirsulfat, Isoniazid, Isosorbidmononitrat, Lactobionat, Lamivudin, Levamisolhydrochlorid, Levofloxacin, Lisinopril, Losartan-Kalium, Metforminhydrochlorid, Methylphenidat, Methylphenidathydrochlorid, Metoprololtartrat, Minocyclinhydrochlorid, Montelukast-Natrium, Naxopren-Natrium, Neostigminbromid, Nicotinamid, Niacin, Nifurtimox, Nortriptylinhydrochlorid, Olanzepin, Oxybutyninchlorid, Penicillamin, Penicillin V-Kalium, Phenytoin-Natrium, Phenformin, Pramipexol, Pravastatin-Natrium, Kaliumchlorid, Primaquinphosphat, Promethazin, Promethazinhydrochlorid, Propanololhydrochlorid, Propoxyphenhydrochlorid, Pseudoephedrinhydrochlorid, Pyridostigminbromid, Pyridoxinhydrochlorid, Quniaprilhydrochlorid, Quetiapin, Ramipril, Ranitidinhydrochlorid, Reboxetin, Risedronat-Natrium, Rosiglitazonmaleat, Sildenafil, Natriumvalproat, Salbutamolsulfat, Stavudin, Sumanirol, Sumatriptansuccinat, Terazosinhydrochlorid, Tetracyclinhydrochlorid, Timololmaleat, Tramadolhydrochlorid, Valacyclovirhydrochlorid, Vancomycin, Venlafaxinhydrochlorid, Verapamilhydrochlorid, Warfarin-Natrium oder Kombinationen davon.

4. Die Dosierform gemäß irgendeinem der vorhergehenden Ansprüche, wobei der Matrixkern von etwa 2 bis etwa 25 Gew.-% wasserlösliches pharmazeutisches Mittel, bis zu etwa 5 Gew.-% Schmiermittel, bis zu etwa 75 Gew.-% Füllstoff, bis zu etwa 25 Gew.-% Freisetzungsmodifikator und bis zu etwa 10 Gew.-% Gleitmittel enthält.

5. Die Dosierform nach irgendeinem der vorhergehenden Ansprüche, wobei die modifizierte Freisetzungsbeschichtung ferner einen Weichmacher enthält.

6. Die Dosierform nach irgendeinem der vorhergehenden Ansprüche, wobei die modifizierte Freisetzungsbeschichtung von etwa 5 bis etwa 70 Gew.-% hydrophobes Polymer, von etwa 30 bis 95 Gew.-% hydrophiles porenbildendes Mittel, bis zu etwa 40 Gew.-% Weichmacher und bis zu 50 Gew.-% Antihaftmittel enthält.

7. Die Dosierform nach irgendeinem der vorhergehenden Ansprüche, wobei das Gewichtsverhältnis des hydrophoben Polymers zu hydrophilem porenbildendem Mittel von etwa 1:1 bis etwa 9:1 beträgt.

8. Die Dosierform nach irgendeinem der vorhergehenden Ansprüche, wobei die Barrierebeschichtung ein wasserlösliches Polymer enthält.

9. Die Dosierform nach Anspruch 8, wobei das wasserlösliche Polymer umfasst:
Hypromellose, Hypromellosephtalat, Methylcellulose, Carboxymethylcellulose-Natrium, Hydroxyethylcellulose, Hydroxypropylcellulose, Carboxymethylhydroxyethylcellulose, Ethylhydroxyethylcellulose, Ethylmethylcellulose, Hydroxyethylmethylcellulose, Hydroxymethylcellulose, Hydroxymethylpropylcellulose, Polyvinylalkohol, Polyvinylpyrrolidon, ein Polyalkylenoxid, ein Polyalkylenglykol, ein Acrylsäurepolymer, ein Vinylacetatcopolymer, ein Polysaccharid, ein Methacrylsäurecopolymer, oder ein Maleinanhydridmethylvinylethercopolymer.

10. Die Dosierform nach irgendeinem der vorhergehenden Ansprüche, wobei die Barrierebeschichtung mit eineem Gewichtszuwachs von etwa 2 bis etwa 6 Gew.-%, basierend auf dem Gesamtgewicht des Matrixkerns, zu Buche schlägt.

11. Die Dosierform nach irgendeinem der vorhergehenden Ansprüche, welche ferner einen Überzug umfasst, welcher die modifizierte Freisetzungsbeschichtung umgibt, wobei der Überzug ein pharmazeutisches Mittel und ein wasserlösliches Polymer enthält.

12. Die Dosierform nach irgendeinem der vorhergehenden Ansprüche in Form einer Tablette.

13. Ein Verfahren zum Herstellen einer festen Dosierform, welches umfasst:
Mischen zwischen ungefähr 10% und etwa 50% eines nicht quellbaren hydrophoben Materials, welches wenigstens ein Polymer enthält, von etwa 2 bis 25 Gew.-% eines wasserlöslichen pharmazeutischen Mittels, eines optionalen Füllstoffs, eines optionalen Schmiermittels und eines optionalen Gleitmittels, um eine Mischung zu bilden;
Komprimieren der Mischung, um einen Matrixkern zu bilden;
Beschichten des Matrixkerns, um eine Barrierebeschichtung zu bilden, welche den Matrixkern umgibt;
Trocknen der Barrierebeschichtung;
Beschichten des barrierebeschichteten Matrixkerns mit einer modifizierten Freisetzungsbeschichtung; und
Trocknen der modifizierten Freisetzungsbeschichtung, um die feste Dosierform zu bilden, wobei die Barrierebeschichtung ein wasserlösliches Polymer enthält, die modifizierte Freisetzungsbeschichtung ein hydrophobes Polymer und ein hydrophiles porenbildendes Mittel enthält und die feste Dosierform in der Lage ist, das pharmazeutische Mittel mit einer Geschwindigkeit nullter Ordnung für eine Dauer von wenigstens vier Stunden nach Verabreichung an einen Probanden freizusetzen.

14. Das Verfahren nach Anspruch 13, wobei das Verfahren weiter die Schritte des Beschichtens der festen Dosierform mit einem Überzug umfasst, wobei der Überzug ein wasserlösliches Polymer und ein pharmazeutisches Mittel enthält.

15. Das Verfahren nach Anspruch 13 oder 14, wobei die feste Dosierform nach irgendeinem der Ansprüche 1 bis 12 hergestellt wird.

## Revendications

1. Forme galénique solide comprenant:
un noyau formant matrice qui comprend entre environ 10% et environ 50% d'une matière hydrophobe non gonflable comprenant au moins un polymère et un agent pharmaceutique soluble dans l'eau; et
un enrobage pour libération modifiée entourant le noyau formant matrice, où l'enrobage pour libération modifiée comprend un polymère hydrophobe et un agent porogène hydrophile;
un enrobage formant barrière soluble dans l'eau entre l'enrobage pour libération modifiée et le noyau formant matrice de telle sorte que l'enrobage formant barrière entoure le noyau formant matrice et l'enrobage pour libération modifiée entoure l'enrobage formant barrière;
où la forme galénique solide est capable de libérer l'agent pharmaceutique à une vitesse de libération d'ordre zéro pendant une durée d'au moins quatre heures après l'administration à un sujet.

2. Forme galénique selon la revendication 1 où la forme galénique solide est capable de libérer l'agent pharmaceutique à une vitesse de libération d'ordre zéro pendant d'environ six à environ dix heures après l'administration à un sujet.

3. Forme galénique selon l'une quelconque des revendications précédentes où l'agent pharmaceutique soluble dans l'eau comprend le sulfate d'abacavir, l'acyclovir, l'acide aminocaproïque, l'alendronate sodium, le chlorhydrate d'amitriptyline, l'amphétamine, l'acétaminophène, l'allopurinol, l'amoxicilline, l'aténolol, le sulfate d'atropine, l'azithromycine, le balsalazide, le chlorhydrate de benzepril, le fumarate de bisoprolol, le chlorhydrate de bupropion, la buformine, le calacyclovir, le captopril, le cefprozil, le chlorhydrate de cétrizine, la cimétidine, la ciprofloxacine, la clindamycine, le maléate de chlorphéniramine, le chlorhydrate de chlorpromazine, le chlorhydrate de clomipramine, le chlorhydrate de clonidine, le bisulfate de clopidogrel, la cloxacilline sodium, le phosphate de codéine, les colchicines, le cyclophosphamide, le citrate de diéthylcarbamazine, le diltiazem, la doxycycline, la doxépine, la DL-méthionine, l'éprosartan, le chlorhydrate d'éthembutol, l'éthosuximide, l'érythromycine, la fexofénadine, le sulfate ferreux, le chlorhydrate de fluoxétine, la fluvastatine, le fosonopril sodium, la gabapentine, le chlorhydrate d'hydralazine, le bitartrate d'hydrocodone, le chlorhydrate d'hydroxyzine, l'hydroxyurée, le sulfate d'indinavir, l'isoniazide, le mononitrate d'isosorbide, le lactobionate, la lamivudine, le chlorhydrate de lévamisole, la lévofloxacine, le lisinopril, le losartan potassium, le chlorhydrate de metformine, le méthylphénidate, le chlorhydrate de méthylphénidate, le tartrate de métoprolol, le chlorhydrate de minocycline, le montelukast sodium, le naproxène sodium, le bromure de néostigmine, le nicotinamide, la niacine, le nifurtimox, le chlorhydrate de nortriptyline, l'olanzépine, le chlorure d'oxybutynine, la pénicillamine, la pénicilline V potassium, la phénytoïne sodium, la phenformine, le pramipexole, la pravastatine sodium, le chlorure de potassium, le phosphate de primaquine, la prométhazine, le chlorhydrate de prométhazine, le chlorhydrate de propranolol, le chlorhydrate de propoxyphène, le chlorhydrate de pseudophédrine, le bromure de pyridostigmine, le chlorhydrate de pyridoxine, le chlorhydrate de quinapril, la quetiapine, le ramipril, le chlorhydrate de ranitidine, la reboxétine, le risédronate sodium, le maléate de rosiglitazone, le sildenafil, le valproate de sodium, le sulfate de salbutamol, la stavudine, le sumanirole, le succinate de sumatriptan, le chlorhydrate de térazosine, le chlorhydrate de tétracycline, le maléate de timolol, le chlorhydrate de tramadol, le chlorhydrate de valacyclovir, la vancomycine, le chlorhydrate de venlafaxine, le chlorhydrate de vérapamil, la warfarine sodium ou leurs combinaisons.

4. Forme galénique selon l'une quelconque des revendications précédentes où le noyau formant matrice comprend d'environ 2 à environ 25 % en poids d'agent pharmaceutique soluble dans l'eau, jusqu'à environ 5 % en poids de lubrifiant, jusqu'à environ 75 % en poids de charge, jusqu'à environ 25 % en poids de modificateur de libération, et jusqu'à environ 10 % en poids d'agent de glissement.

5. Forme galénique selon l'une quelconque des revendications précédentes où l'enrobage pour libération modifiée comprend en outre un plastifiant.

6. Forme galénique selon l'une quelconque des revendications précédentes où l'enrobage pour libération modifiée comprend d'environ 5 à environ 70 % en poids de polymère hydrophobe, d'environ 30 à environ 95 % en poids d'agent porogène hydrophile, jusqu'à environ 40 % en poids de plastifiant et jusqu'à 50 % en poids d'agent antipégosité.

7. Forme galénique selon l'une quelconque des revendications précédentes où le rapport en poids du polymère hydrophobe à l'agent porogène hydrophile est d'environ 1:1 à environ 9:1.

8. Forme galénique selon l'une quelconque des revendications précédentes où l'enrobage formant barrière comprend un polymère soluble dans l'eau.

9. Forme galénique selon la revendication 8 où le polymère soluble dans l'eau comprend l'hypromellose, le phtalate d'hypromellose, la méthylcellulose, la carboxyméthylcellulose sodium, l'hydroxyéthylcellulose, l'hydroxypropylcellulose, la carboxyméthylhydroxyéthylcellulose, l'éthylhydroxy-éthylcellulose, l'éthylméthylcellulose, l'hydroxyéthylméthylcellulose, l'hydroxy-méthylcellulose, l'hydroxyméthylpropylcellulose, le poly(alcool vinylique), la polyvinylpyrrolidone, un poly(oxyde d'alkylène), un polyalkylèneglycol, un polymère d'acide acrylique, un copolymère d'acétate de vinyle, un polysaccharide, un copolymère d'acide méthacrylique ou un copolymère anhydride maléique/méthylvinyléther.

10. Forme galénique selon l'une quelconque des revendications précédentes où l'enrobage formant barrière est présent à un gain de poids d'environ 2 à environ 6 % en poids sur la base du poids total du noyau formant matrice.

11. Forme galénique selon l'une quelconque des revendications précédentes comprenant en outre un enrobage supérieur entourant l'enrobage pour libération modifiée, où l'enrobage supérieur comprend un agent pharmaceutique et un polymère soluble dans l'eau.

12. Forme galénique selon l'une quelconque des revendications précédentes sous forme d'un comprimé.

13. Procédé de production d'une forme galénique solide comprenant:
le mélange d'entre environ 10% et environ 50% d'une matière hydrophobe non gonflable comprenant au moins un polymère, d'environ 2 à 25 % en poids d'un agent pharmaceutique soluble dans l'eau, d'une charge éventuelle, d'un lubrifiant éventuel et d'un agent de glissement éventuel pour former un mélange;
la compression du mélange pour former un noyau formant matrice;
l'enrobage du noyau formant matrice pour former un enrobage formant barrière entourant le noyau formant matrice;
le séchage de l'enrobage formant barrière;
l'enrobage du noyau formant matrice muni de l'enrobage formant barrière avec un enrobage pour libération modifiée; et
le séchage de l'enrobage pour libération modifiée pour former la forme galénique solide, où l'enrobage formant barrière comprend un polymère soluble dans l'eau, l'enrobage pour libération modifiée comprend un polymère hydrophobe et un agent porogène hydrophile, et la forme galénique solide est capable de libérer l'agent pharmaceutique à une vitesse d'ordre zéro pendant une durée d'au moins quatre heures après l'administration à un sujet.

14. Procédé selon la revendication 13 où le procédé comprend en outre les étapes d'enrobage de la forme galénique solide avec un enrobage supérieur, où l'enrobage supérieur comprend un polymère soluble dans l'eau et un agent pharmaceutique.

15. Procédé selon la revendication 13 ou 14 où la forme galénique solide selon l'une quelconque des revendications 1-12 est produite.
